# EUROPÄISCHE PATENTSCHRIFT

(11) **EP 2 687 239 B1**
(45) Veröffentlichungstag und Bekanntmachung des Hinweises auf die Patenterteilung: **06.03.2019**
(21) Anmeldenummer: 13176484.7
(22) Anmeldetag: 15.07.2013
(51) Int. Cl.: A61L 24/02, A61L 27/44, A61L 24/00

(54) **Pastenförmiger Knochenzement**
Bone cement in paste form
Ciment osseux pâteux

(30) Priorität: 20.07.2012 DE 102012014418
(43) Veröffentlichungstag der Anmeldung: 22.01.2014
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A2- 2 550 980
- WO-A1-2011/068481
- WO-A1-2011/141889
- WO-A2-01/49327
- DE-A1-102010 024 653
- US-A1- 2006 030 637
- DATABASE WPI Week 200509 Thomson Scientific, London, GB; AN 2005-081022 XP002753085, & WO 2004/103420 A1 (CONSEJO SUPERIOR INVESTIGACIONES CIENTIF) 2. Dezember 2004 (2004-12-02) -& WO 2004/103420 A1 (CONSEJO SUPERIOR INVESTIGACION [ES]; MENDEZ GONZALEZ JOSE ALBERTO [ES]) 2. Dezember 2004 (2004-12-02)

## Beschreibung

Die vorliegende Erfindung betrifft einen Kit, oder eine Paste, hergestellt aus einem Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie, sowie einen Formkörper.

Konventionelle Polymethylmethacrylat-Knochenzemente (PMMA-Knochenze-mente) sind seit Jahrzehnten bekannt und gehen auf die grundlegenden Arbeiten von Sir Charnley zurück (Charnley, J.: "Anchorage of the femoral head prosthesis of the shaft of the femur"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4,015,945, EP-A-0 674 888 und JP-A-2003181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmethacrylatknochenzementen wurden pastenförmige Polymethylmethacrylatknochen-zemente in den Offenlegungsschriften DE-A-10 2007 052 116, DE-A-10 2007 050 762 und DE-A-10 2007 050 763 beschrieben, welche ein radikalisch polymerisierbares Methacrylat-Monomer, ein in diesem Methacrylat-Monomer lösliches Polymer und - ein in diesem Methacrylat-Monomer unlösliches partikuläres Polymer beinhalten. Solche pastenförmigen Polymethylmethacrylat-Knochenzemente können als Einkomponenten-System (in diesem Fall beinhaltet die Paste alle zur Aushärtung notwendigen Komponenten, insbesondere einen aktivierbaren radikalischen Initiator, z. B. einen Photoinitiator oder ein Photoiniatorsystem) oder als Zweikomponenten-System (in diesem Fall umfasst das System zwei vorgemischte, lagerstabile Pasten, von denen eine einen radikalischen Polymerisationsinitiator und die andere einen Polymerisationsaktivator aufweist) vorliegen. Im Falle der Zweikomponenten-Systeme wird weiterhin zwischen einem "symmetrischen System" (in diesem Fall beinhalten beide Pasten ein in dem Methacrylat-Monomer unlösliches partikuläres Polymer) und "unsymmetrischen Systemen" (in diesem Fall beinhaltet nur eine der beiden Pasten ein in dem Methacrylat-Monomer unlösliches partikuläres Polymer) unterschieden.

Der aus den vorstehen beschriebenen Pasten hergestellte Knochenzement weist als Folge der gewählten Zusammensetzung eine ausreichend hohe Viskosität und Kohäsion auf, um dem Blutungsdruck bis zur Aushärtung standzuhalten. Durch die fortschreitende Polymerisation härtet die Paste unter Verbrauch der Methacrylat-Monomere aus.

Bei den in DE-A-10 2007 052 116, DE-A-10 2007 050 762 und DE-A-10 2007 050 763 offenbarten pastenförmigen Polymethylmethacrylat-Knochenzementen sind neben wenigstens einem radikalisch polymerisierbaren Monomer und mindestens einem darin gelösten Polymer in dem Monomer unlösliche Polymerpartikel enthalten. Diese unlöslichen Polymerpartikel stellen einen Füllstoff dar. Dieser beeinflusst die Viskosität der Zementpasten erheblich. Die Polymerpartikel sind essentiell für die Verarbeitungseigenschaften, damit die Zementpasten während der Applikationsphase bei der Formgebung möglichst nur eine minimale elastische Rückstellbewegung zeigen. Dadurch lassen sich die Zementpasten in der Verarbeitungsphase in beliebige Gestalt formen, wie es von konventionellen Polymethylmethacrylat-Knochenzementen, die auf der Vermischung von Polymerpulver und Monomerflüssigkeit beruhen, allgemein bekannt ist.

In Methacrylatmonomeren unlösliche, vernetzte Polymerpartikel sind in der Herstellung relativ aufwendig und daher teuer. Daher ist es wünschenswert, ein alternatives, kostengünstiges, partikuläres Material zu finden, das nach Zusatz zu Gemischen aus Methacrylatmonomeren und darin gelösten Polymeren, in ähnlicher Weise wie vernetzte Polymerpartikel, Pasten ergibt, die nach Formgebung nur ein minimales elastisches Rückstellvermögen zeigen.

Problematisch ist jedoch, dass bisher verwendeten vernetzten Polymerpartikel auch einen Beitrag zur mechanischen Stabilität der ausgehärteten pastenförmigen Zemente lieferten. Es kommt deshalb darauf an, einen alternativen Füllstoff zu finden, der einerseits die erforderlichen Verarbeitungseigenschaften der Pasten gewährleistet und andererseits die mechanischen Parameter der ausgehärteten Zemente nicht negativ beeinflusst, so dass die Anforderungen der ISO 5833 hinsichtlich der mechanischen Stabilität erfüllt werden.

In DE102010024653 wird ein Knochenzement-Kit auf der Basis zweier Pasten beschrieben.

Der vorliegenden Erfindung lag die Aufgabe zugrunde, die sich aus dem Stand der Technik ergebenden Nachteile im Zusammenhang mit auf Pasten basierenden Knochenzement-Systemen, insbesondere im Zusammenhang mit den vorstehend beschriebenen Zweikomponenten-Systemen, zu überwinden.

Insbesondere lag der vorliegenden Erfindung die Aufgabe zugrunde, eine auf einem Zweikomponenten-System basierenden Knochenzementpaste bereitzustellen, die im Vergleich zu den aus den Stand der Technik bekannten Knochenzementpasten aus kostengünstigeren Ausgangsmaterialien hergestellt werden kann, die aber dennoch die gleichen Verarbeitungseigenschaften aufweist wie die Pasten des Standes der Technik.

Einen Beitrag zur Lösung der eingangs genannten Aufgaben leistet eine Paste (nicht erfindungsgemäß) beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikulärer anorganischer Füllstoff mit einer BET-Oberfläche von mindestens 40 m²/g, besonders bevorzugt von mindestens 200 m²/g und am meisten bevorzugt von mindestens 300 m²/g ist.

Beschrieben wird der gegenüber den bisher bekannten pastenförmigen Polymethylmethacrylat-Knochenzementen überraschende Befund, dass sich gut formbare, modellierbare Knochenzementpasten unter Verwendung von partikulären, anorganischen Füllstoffen, die eine BET-Oberfläche von mindestens 40 m²/g besitzen, herstellen lassen. Überraschend ist, dass die bisher üblichen in Methacrylat-Monomeren unlöslichen, vernetzten Polymerpartikeln ganz bzw. teilweise durch partikulären, anorganischen Füllstoffen, die eine BET-Oberfläche von mindestens 40- m²/g besitzen, ersetzt werden können. Anorganische Füllstoffe dieser Art sind deutlich preiswerter als vernetzte Polymerpartikel und können dadurch wirtschaftlich vorteilhaft zur Herstellung von pastenförmigen Polymethylmethacrylat-Knochenzementen verwendet werden.

Es gelang überraschend trotz der Verwendung von anorganischen Partikeln anstelle von vernetzten Polymerpartikeln Zementpasten herzustellen, die nach erfolgter Aushärtung die mechanischen Anforderungen der ISO 5833 erfüllen.

Besonders vorteilhaft lassen sich unter Verwendung von partikulären, anorganischen Füllstoffen, die eine BET-Oberfläche von mindestens 40- m²/g besitzen, pastenförmige Knochenzementpasten für die Kyphoplastie und die Vertebroplastie herstellen, die einen hohen Anteil an Röntgenopakern enthalten und eine Verarbeitungszeit von mindestens 15 Minuten besitzen.

Grundsätzlich kann die Paste ein vorstehend beschriebenes Einkomponenten-System sein oder durch Vermischen der beiden Pasten eines vorstehend beschriebenen Zweikomponenten-Systems erhalten werden.

Die Paste beinhaltet als eine Komponente wenigstens ein radikalisch polymerisierbares Monomer, wobei es sich vorzugsweise um ein Methacrylat-Monomer, insbesondere um ein Methacrylat-Monomer handelt, welches bei einer Temperatur von 25°C und einem Druck von 1.013 hPa flüssig ist.

Vorzugsweise handelt es sich bei dem radikalisch polymerisierbaren Monomer nicht um einen von Bisphenol A abgeleiteten Methacrylsäureester.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestern kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Das eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung des radikalisch polymerisierbaren Monomers einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

Vorzugsweise beinhaltet die Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste.

Als weitere Komponente beinhaltet die Paste wenigstens ein in dem wenigstens einen radikalisch polymerisierbaren Monomer lösliches Polymer. Ein Polymer ist in dem polymerisierbaren Monomer löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse von wenigstens 150.000 g/mol. Beispielsweise kann es sich bei dem löslichen Polymer um ein Polymer oder Copolymer eines Methacrylsäureesters handeln. Gemäß einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in der Paste liegt üblicherweise in einem Bereich von 1 bis 85 Gew.-%, bezogen auf das Gesamtgewicht der Paste.

Weiterhin beinhaltet die Paste wenigstens einen in dem wenigstens einen radikalisch polymerisierbaren Monomer schwer- oder unlöslichen Füllstoff, wobei der Füllstoff ein partikulärer anorganischer Füllstoff mit einer BET-Oberfläche von mindestens 40 m²/g, besonders bevorzugt von mindestens 200 m²/g und am meisten bevorzugt von mindestens 300 m²/g ist.

Die BET-Messung ist ein Analyseverfahren zur Charakterisierung von Oberflächen von Festkörpern mit Hilfe der Gasadsorption. Die Bestimmungsmethode ist in der DIN ISO 9277:2003-05 (Bestimmung der spezifischen Oberfläche von Feststoffen durch Gasadsorption nach dem BET-Verfahren) beschrieben.

Gemäß einer bevorzugten Ausgestaltung der Paste handelt es sich bei dem partikulären anorganischen Füllstoff um einen partikulären, anorganischen Füllstoff, der kovalent an den Partikeln kovalent gebundene Hydroxylgruppen aufweist. Durch diese an der Partikeloberfläche angeordneten Hydroxylgruppen können sich Wasserstoffbrückenbindungen zwischen den Füllstoffpartikeln ausbilden, die durch Einwirkung von mechanischer oder thermischer Energie reversibel gelöst werden können. Ganz besonders bevorzugt sind partikuläre, anorganische Füllstoffe, die Silanol-Gruppen (HO-Si-Gruppen) aufweisen.

Bevorzugt ist in diesem Zusammenhang, dass der partikuläre, anorganische Füllstoff ausgewählt ist aus der Gruppe bestehend aus pyrogenem Siliziumdioxid, pyrogenen Metall-Siliziummischoxiden, Titandioxid, Bentonit, Montmorillonit und einer Mischung aus mindestens zwei davon.

Daneben ist es auch möglich, hydrophobiertes pyrogenes Siliziumdioxid einzusetzen. Die Herstellung des hydrophoben Siliziumdioxids kann nach dem Stand der Technik durch Behandlung von pyrogenem Siliziumdioxid mit Dialkyldichlorsilanen (z. B. Dimethyldichlorsilan) erfolgen.

Ganz besonders bevorzugt als partikulärer, anorganischer Füllstoff ist pyrogenes Siliziumdioxid mit einer BET-Oberfläche von mindestens 40 m²/g, besonders bevorzugt von 200 m²/g und am meisten bevorzugt von 300 m²/g. Derartiges pyrogenes Siliziumdioxid ist unter anderem unter dem Markennamen Aerosil® mit spezifischen BET-Oberflächen von 50 m²/g, 90 m²/g, 200 m²/g und 380 m²/g kommerziell verfügbar.

Anstelle von pyrogenem Siliziumdioxid sind auch Mischoxide des Siliziums mit Oxiden von Metallen geeignet. So eigenen sich auch Mischoxide aus Silizium und Eisen.

Die Menge des in dem partikulären, anorganischen Füllstoff in der Paste liegt üblicherweise in einem Bereich von 0,5 bis 25 Gew.-%, besonders bevorzugt von 1 bis 20 Gew.-% und am meisten bevorzugt in einem Bereich von 5 bis 15 Gew.-%, jeweils bezogen auf das Gesamtgewicht der Paste. Gegebenenfalls kann die Paste neben dem vorstehend beschriebenen partikulären, anorganischen Füllstoff auch noch bestimmte Mengen eines anderen Füllstoffes, beispielsweise die aus dem Stand der Technik bekannten, vernetzten Polymerpartikel, beinhalten, wobei in diesem Falle das Gewichtsverhältnis von partikulären, anorganischen Füllstoff zu den vernetzten Polymerpartikeln vorzugsweise mindestens 1 : 15 (also mindestens etwa 6 Gew.-% partikulärer, anorganischer Füllstoff bezogen auf die Gesamtmenge an Füllstoff), ganz besonders bevorzugt mindestens 1 : 1 (also mindestens 50 Gew.-% partikulärer, anorganischer Füllstoff bezogen auf die Gesamtmenge an Füllstoff) beträgt.

Vorzugsweise ist die Paste spätestens 15 Minuten nach ihrer Herstellung klebfrei nach ISO 5833.

Die Paste kann weiterhin wenigstens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder wenigstens einen Polymerisations-initiator, wenigstens einen Polymerisationsbeschleuniger und gegebenenfalls wenigstens einen Co-Polymerisationsbeschleuniger beinhalten.

Im Falle eines Einkomponenten-Systems handelt es sich bei dem Polymerisationsinitiator vorzugsweise um einen aktivierbaren Polymerisationsinitiator, z. B. um einen in der Paste gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems die Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Im Falle einer Paste, welche durch die Kombination zweier Pasten eines Zweikomponenten-Systems erhalten wurde, beinhaltet diese Paste vorzugsweise wenigstens einen Polymerisationsinitiator (der in der einen Paste des Zweikomponenten-Systems enthalten war) und wenigstens einen Polymerisationsbeschleuniger (der in der anderen Paste des Zweikomponenten-Systems enthalten war).

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden Verbindungen verstanden, die wenigstens eine Peroxogruppe (-O-O-)enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Gemäß einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amyl-hydroperoxid, Di-Isopropylbenzen-mono-hydroperoxid und einer Mischung aus mindestens zwei davon besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten. Gemäß einer besonderen Ausgestaltung der Paste ist das Barbitursäurederivat ausgewählt ist aus der Gruppe bestehend aus 1,5-disubstituierten Barbituraten-und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Bevorzugt sind Barbiturate, die an Position 1 und an Position 5 jeweils einen Substituenten oder an den Positionen 1, 3 und 5 jeweils einen Substituenten -tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt.

Bevorzugte Schwermetallverbindungen sind ausgewählt aus der Gruppe bestehend Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der Paste ist der Polymerisationsbeschleuniger ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäurediimid und einer Mischung aus mindestens zwei davon.

Eine weitere vorteilhafte Ausgestaltung besteht in Verwendung von Kombinationen aus Schwermetallsalzen und mindestens einem Vertreter aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei sind Zweifachkombinationen und auch Dreifachkombinationen von unterschiedlichen Polymerisationsbeschleunigern im Rahmen der Erfindung.

Eine vorteilhafte Ausgestaltung besteht darin, dass gegebenenfalls wenigstens ein Co-Polymerisationsbeschleuniger in der Paste enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin, 1,8-Diazabicyclo[5.4.0-]undec-7-en und 1,5-Diazabicyclo(4.3.0)-non-5-en als Co-Akzeleratoren bevorzugt sind.

Die Paste kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht der Paste, beinhalten.

Die Paste kann neben den vorstehend genannten Komponenten weitere Bestandteile beinhalten.

Gemäß einer bevorzugten Ausführungsform der Paste kann diese wenigstens ein Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentration an Röntgenopaker, insbesondere die Konzentration an Zirkoniumdioxid, in der Paste kann in beispielsweise in einem Bereich von 3 bis 30 Gew.-% liegen.

Gemäß einer weiteren bevorzugten Ausführungsform kann die Paste wenigstens einen Farbstoff beinhalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform kann die Paste wenigstens einen pharmazeutischen Wirkstoff beinhalten. Der wenigstens eine pharmazeutische Wirkstoff kann in der Paste in gelöster oder suspendierter Form enthalten sein. Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum. Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht. Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht. Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht. Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht. Das wenigstens eine Hormon ist vorzugsweise aus der Gruppe ausgewählt, die aus Serotonin, Somatotropin, Testosteron und Östrogen besteht. Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht. Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht. Das wenigstens eine Bisphosphonat ist vorzugsweise aus der Gruppe ausgewählt, die aus Zoledronat und Aledronat besteht.

Gemäß einer weiteren bevorzugten Ausführungsform kann die Paste wenigstens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die Paste wenigstens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die Paste wenigstens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Einen Beitrag zur Lösung der eingangs genannten Aufgabe leistet auch ein Knochenzement-Kit (erfindungsgemäß), aufweisend eine Paste A und eine Paste B,
wobei
(a) Paste A
   (a1) wenigstens ein radikalisch polymerisierbares Monomer,
   (a2) wenigstens ein in (a1) lösliches Polymer in einem Bereich von 3 bis 16 Gew.-% in Bezug auf das Gesamtgewicht der Paste, und
   (a3) wenigstens einen Polymerisationsinitiator aus der Gruppe der Peroxide in einer Menge in einem Bereich von 0,01 bis 10 Gew.-%, bezogen auf das Gesamtgewicht der Paste A beinhaltet;
(b) Paste B
   (b1) wenigstens ein radikalisch polymerisierbares Monomer,
   (b2) wenigstens ein in (b1) lösliches Polymer in einem Bereich von 25 bis 85 Gew.-% in Bezug auf das Gesamtgewicht der Paste, und
   (b3) wenigstens einen Polymerisationsbeschleuniger beinhaltet;
   und wobei mindestens eine der Pasten A und B als Komponente (a4) bzw. (b4) wenigstens einen in (a1) bzw. (b1) schwer- oder unlöslichen Füllstoff beinhaltet, wobei der Füllstoff ein partikulärer anorganischer Füllstoff mit einer BET-Oberfläche von mindestens 40 m²/g, besonders bevorzugt von mindestens 200 m²/g und am meisten bevorzugt von mindestens 300 m²/g ist, und wobei Paste A 1 bis 50 Gew.-%, bezogen auf das Gesamtgewicht der Paste A, des Füllstoffs (a4) und Paste B weniger als 5 Gew.-%, bezogen auf das Gesamtgewicht der Paste B, des Füllstoffs (b4) beinhaltet.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Vorzugsweise ist der Kit als Vorrichtung zur Herstellung von Knochenzement ausgestaltet, die einen ersten Behälter und einen zweiten Behälter aufweist, wobei der erste Behälter die Paste A und der zweite Behälter die Paste aufweist, wobei wenigstens einer der Behälter zu öffnen ist, um nach dem Öffnen ein Vermischen der Pasten A und B zu gestatten, und eine Mischeinheit zum Vermischen der Pasten A und B.

Als radikalisch polymerisierbares Monomer (a1) bzw. (b1), als in (a1) bzw. (b1) lösliches Polymer (a2) bzw. (b2), als Polymerisationsinitiator (a3), als Polymerisationsbeschleuniger (b3) und als partikulärer anorganischer Füllstoff (a4) bzw. (b4) sind diejenigen Komponenten bevorzugt, die bereits eingangs im Zusammenhang mit der Paste als bevorzugtes radikalisch polymerisierbares Monomer, als in diesem Monomer lösliches Polymer, als Polymerisationsinitiator, als Polymerisationsbeschleuniger und als partikulärer anorganischer Füllstoff beschrieben wurden.

Vorzugsweise beinhalten die Paste A und die Paste B das wenigstens eine radikalisch polymerisierbare Monomer (a1) bzw. (b1) in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste A bzw. der Paste B.

Vorzugsweise beinhaltet die Paste A den Polymerisationsinitiator (a3) in einer Menge ein einem Bereich von 0,01 bis 10 Gew.-%, mehr bevorzugt in einem Bereich von 0,01 bis 8 Gew.-% und noch mehr bevorzugt in einem Bereich von 0,01 bis 5 Gew.-%, jeweils bezogen auf das Gesamtgewicht der Paste A.

Sofern es sich bei dem Polymerisationsbeschleuniger (b3) um eine Schwermetallverbindung handelt, die aus der Gruppe bestehend aus Schwermetallsalzen und Schwermetallkomplexen ausgewählt ist, insbesondere jedoch um eine Schwermetallverbindung, die ausgewählt ist aus der Gruppe bestehend aus Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon, so beinhaltet die Paste B einen solchen Polymerisationsbeschleuniger (b3) vorzugsweise in einer Menge in einem Bereich von 0,0005 bis 0,5 Gew.-%, bezogen auf das Gesamtgewicht der Paste B.

Sofern es sich bei dem Polymerisationsbeschleuniger (b3) um eine Verbindung ausgewählt ist aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en, 1,5-Diazabicyclo(4.3.0)non-5-en, Phthalimid, Maleimid, Succinimid, Pyromelithsäurediimid und eine Mischung aus mindestens zwei davon handelt, so beinhaltet die Paste B einen solchen Polymerisationsbeschleuniger (b3) vorzugsweise in einer Menge in einem Bereich von 0,1 bis 10 Gew.-%, bezogen auf das Gesamtgewicht der Paste B.

Insbesondere die Paste A kann weiterein als Komponente (a5) den vorstehend beschriebenen Co-Polymerisationsbeschleuniger beinhalten, bei dem es sich vorzugsweise um eine Verbindung ausgewählt ist aus der Gruppe bestehend aus N,N-Dimethyl-p-toluidin, N,N-Bis-hydroxyethyl-p-toluidin, N,N-Dimethyl-anilin, 1,8-Diazabicyclo[5.4.0]undec-7-en, 1,5-Diazabi-cyclo(4.3.0)non-5-en und einer Mischung aus mindestens zwei davon handelt. In diesem Zusammenhang ist es bevorzugt, dass die Paste A den wenigstens einen Co-Polymerisationsbeschleuniger (a5) in einer Menge in einem Bereich von 0,1 bis 10 Gew.-%, bezogen auf das Gesamtgewicht der A, beinhaltet.

Sofern eine der beiden Pasten des erfindungsgemäßen Kits den schwer- oder unlöslichen Füllstoff beinhaltet und die andere Paste überhaupt keinen schwer- oder unlöslichen Füllstoff oder einen schwer- oder unlöslichen Füllstoff in einer im Vergleich zur Menge in der anderen Paste vernachlässigbaren Menge, so handelt es sich um einen sogenannten "asymmetrischen" Kit. Ist hingegen der schwer- oder unlösliche Füllstoff in beiden Pasten in etwa vergleichbarer Menge vorhanden, so handelt es sich um einen sogenannten "symmetrischen" Kit.

Weiterhin können in den Pasten A und/oder B außer den vorstehend beschriebenen Komponenten auch weitere Zusatzstoffe enthalten sein, wie etwa Röntgenopaker, Farbstoffe, pharmazeutische Wirkstoffe, biokompatible Elastomere, Monomere mit Haftgruppen oder Stabilisatoren, wobei auch hier diejenigen Komponenten bevorzugt sind, die bereits eingangs im Zusammenhang mit der erfindungsgemäßen Paste als bevorzugte Röntgenopaker, Farbstoffe, pharmazeutische Wirkstoffe, biokompatible Elastomere, Monomere mit Haftgruppen und Stabilisatoren beschrieben wurden.

Gemäß einer ersten besonderen Ausgestaltung des erfindungsgemäßen Kits handelt es sich bei dem Kit um einen "asymmetrischen" Kit. Die Paste A enthält bevorzugt 1 bis 50 Gew.-%, besonders bevorzugt 1,0 bis 15 Gew.-%, noch mehr bevorzugt 30 bis 55 Gew.-% und am meisten bevorzugt 1 bis 5 Gew.-%, jeweils bezogen auf das Gesamtgewicht der Paste A, des in (a1) unlöslichen Füllstoffs (a4) und die Paste B enthält weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b4), wobei es am meisten bevorzugt ist, dass die Paste B überhaupt keinen in (b1) unlöslichen Füllstoff (b4) beinhaltet.

Weiterhin enthält im erfindungsgemäßen Kits die Paste A das in (a1) lösliche Polymer (a2) in einer Menge in einem Bereich von 3 bis 16 Gew.-%, bezogen auf das Gesamtgewicht der Paste A, und Paste B ein in (b1) lösliches Polymer (b2) in einer Menge in einem Bereich von 25 bis 85 Gew.-%, besonders bevorzugt in einem Bereich von 35 bis 85 Gew.-%, noch mehr bevorzugt in einem Bereich von 35 bis 80 Gew.-% und am meisten bevorzugt in einem Bereich von 35 bis 75 Gew.-%, jeweils bezogen auf das Gesamtgewicht der Paste B, beinhaltet.

Weiterhin ist es im Zusammenhang mit dieser ersten besonderen Ausgestaltung des erfindungsgemäßen Kits bevorzugt, dass das Gewichtsverhältnis von in (b1) unlöslichem Füllstoff (b4) zu dem wenigstens einen in (b1) löslichen Polymer (b2) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es sich bei dem Kit um einen "symmetrischen" Kit. In diesem Zusammenhang ist es bevorzugt, dass die Paste A 0,5 bis 35 Gew.-%, besonders bevorzugt 0,5 bis 15 Gew.-%, jeweils bezogen auf das Gesamtgewicht der Paste A, des in (a1) unlöslichen Füllstoffs (a4) und Paste B 0,5 bis weniger als 5 Gew.-%, jeweils bezogen auf das Gesamtgewicht der Paste B, des in (b1) unlöslichen Füllstoffs (b4) beinhaltet.

Die Paste bzw. der erfindungsgemäße Kit, der wenigstens die Pasten A und B enthält, dient zur Herstellung von Knochenzement. Im Falle des Kits werden dazu die wenigstens zwei Pasten A und B miteinander vermischt, wobei wiederum eine Paste, Paste C, erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B. Das Mischen kann mit üblichen Mischgeräten, beispielsweise einem statischen Mischer oder einem dynamischen Mischer erfolgen.

Nach dem Vermischen der Pasten des Kits ist die letztlich erhaltene Paste C (die der eingangs genannten erfindungsgemäßen Paste entspricht) spätestens nach 15 Minuten nach der Norm ISO 5833 klebfrei.

Der aus der Paste bzw. der aus Paste C durch Aushärtung entstehende Knochenzement erreicht etwa sechs bis acht Minuten nach dem Vermischen der im Kit enthaltenen Pasten eine hohe Festigkeit.

Die Paste bzw. die Paste C, hergestellt aus dem erfindungsgemäßen Kit, kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff *"Spacer"* werden dabei Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus der Paste bzw. dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen Verwendungen von Knochenzement sind aus der Literatur bekannt und dort mannigfach beschrieben.

Einen Beitrag zur Lösung der eingangs genannten Aufgaben leistet auch ein Formkörper, erhältlich durch die Polymerisation einer Paste, erhältlich durch das Vermischen der Paste A und der Paste B des erfindungsgemäßen Kits, oder durch die Polymerisation einer Paste. Formkörper im Sinne der vorliegenden Erfindung können jedwede dreidimensionalen Körper sein, insbesondere die vorstehend beschriebenen "Spacer".

Die Erfindung wird durch die nachstehend beschriebenen Beispiele erläutert, die jedoch die Erfindung nicht einschränken.

### AUSFÜHRUNGSBEISPIELE

Die Pasten A der Beispiele A1-6 wurden durch einfaches Vermischen der Komponenten hergestellt. Die gebildeten Pasten wurden dann über Nacht bei Raumtemperatur gelagert.

**Paste A**

| | | Zusammensetzung der Pasten A | | | | | | | | |
|---|---|---|---|---|---|---|---|---|---|---|
| Beispiel-Nr. | CH [mg] | BH [g] | DM [g] | EG [g] | MA [g] | MMA [g] | PL1 [g] | AE [g] | ZrO₂ [g] | Stab [mg] |
| A1 | 75 | 2,00 | - | - | - | 14,00 | 6,00 | 2,00 | 20,00 | 20 |
| A2 | 50 | 1,80 | 0,20 | - | - | 14,00 | 7,00 | 1,50 | 20,00 | 20 |
| A3 | 50 | 1,80 | 0,20 | 0,50 | - | 13,50 | 7,00 | 1,00 | 20,00 | 20 |
| A4 | 50 | 1,90 | 0,10 | - | - | 14,00 | 6,00 | 1,50 | 20,00 | 20 |
| A5 | 50 | 1,40 | 0,60 | 0,10 | 0,40 | 17,70 | 13,60 | 1,00 | 4,80 | 20 |
| A6 | 50 | 1,40 | 0,60 | 0,10 | 0,40 | 17,70 | 13,60 | 2,00 | 4,80 | 20 |

- CH:: Cumolhydroperoxid
- BH:: N,N-Bis-(2-hydroxyethyl)-p-toluidin
- DM:: N,N-Dimethyl-p-toluidin
- EG:: Ethylenglykoldimethacrylat
- MA:: Methacrylamid
- MMA:: Methylmethacrylat
- PL1:: lineares Poly(methylmethacrylat-co-methylacrylat) Mw < 500.000 g/mol
- AE:: Aerosil® 380 (pyrogene Kieselsäure)
- ZrO₂:: Zirkoniumdioxid
- Stab:: 2,6-Di-t-butyl-4-methyl-phenol

Die Pasten B der Beispiele B1-6 wurden ebenfalls durch einfaches Vermischen der Komponenten hergestellt. Die gebildeten Pasten wurden dann über Nacht bei Raumtemperatur gelagert.

**Paste B**

| | Zusammensetzung der Pasten B | | | | | |
|---|---|---|---|---|---|---|
| Beispiel-Nr. | SAC [g] | CuOct [mg] | MMA [g] | PL1 [g] | AE [g] | Stab [mg] |
| B1 | 1,00 | 55 | 22,00 | 18,00 | - | 35 |
| B2 | 1,00 | 55 | 23,00 | 17,00 | - | 35 |
| B3 | 1,00 | 55 | 23,00 | 17,00 | - | 35 |
| B4 | 1,00 | 55 | 22,00 | 18,00 | 0,50 | 35 |
| B5 | 1,00 | 55 | 21,20 | 17,50 | - | 35 |
| B6 | 1,00 | 55 | 21,20 | 17,50 | - | 35 |

- SAC:: Saccharin
- CuOct:: Kupfer(II)-2-ethylhexanoat
- MMA:: Methylmethacrylat
- PL1:: lineares Poly(methylmethacrylat-co-methylacrylat), Mw < 500.000 g/mol
- AE:: Aerosil® 380 (pyrogene Kieselsäure)
- Stab:: 2,6-Di-t-butyl-4-methyl-phenol

Die Pasten A und B der Beispiele A1-6 und B1-6 wurden im Gewichtverhältnis 1 : 1 miteinander gemischt. Es entstanden sofort klebfreie Pasten C, die im Fall der Pasten C1 bis C4 eine Verarbeitungsphase bis zu 20 Minuten hatten. In dieser Verarbeitungsphase konnten diese Pasten problemlos durch 18 G-Kanülen (Kanüle mit 1,2 mm Außendurchmesser) gepresst werden.

Die Pasten C5 und C6 (Vergleichsbeispiele, nicht erfindungsgemäß) dagegen zeigten eine Viskosität und eine Verarbeitungsphase ähnlich zu konventionellen hoch-viskosen Polymethylmethycrylat-Knochenzementen. Die Verarbeitungsphase dauerte 4-5 Minuten.

Mit den aus den Pasten A und B der Beispiele 1-6 hergestellten gemischten Pasten C (Gewichtsverhältnis 1 zu 1 von Paste A zu Paste B) wurden streifenförmige Prüfkörper mit den Abmessungen (75 mm × 10 mm × 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

| Pasten C | Zusammensetzung der Pasten C | 4-Punkt-Biegefestigkeit | Biege-Modul | DruckFestigkeit |
|---|---|---|---|---|
| | | [MPa] | [MPa] | [MPa] |
| C1 | A1 + B1 | 57,9 ± 0,7 | 2686 ± 51 | 102,0 ± 2,4 |
| C2 | A2 + B2 | 58,9 ± 0,4 | 2643 ± 53 | 96,5 ± 4,1 |
| C3 | A3 + B3 | 58,7 ± 1,9 | 2682 ± 153 | 94,4 ± 4,3 |
| C4 | A4 + B4 | 62,9 ± 1,7 | 2927 ± 91 | 92,2 ± 6,2 |
| C5 | A5 + B5 | 58,1 ± 1,4 | 2405 ± 77 | 91,2 ± 4,4 |
| C6 | A6 + B6 | 60,1 ± 3,1 | 2561 ± 192 | 93,9 ± 3,3 |

Die Ergebnisse der Prüfung der 4-Punkt-Biegefestigkeit, des Biegemoduls und der Druckfestigkeit der ausgehärteten Pasten C1-6 (Pasten C5 und C6 sind nicht erfindungsgemäße Vergleichsbeispiele) zeigen, dass die Anforderungen der ISO 5833 hinsichtlich der mechanischen Festigkeit erfüllt werden. Die ISO 5833 schreibt folgende Parameter vor: eine 4-Punkt-Biegefestigkeit von mindestens 50 MPa, ein Biegemodul von mindestens 1800 MPa und eine Druckfestigkeit von mindestens 70 MPa.

Außerdem wurden weitere Pasten B analog der Paste B6, jedoch mit einem Zusatz von jeweils 1,0 g Vancomycinhydrochlorid, Clindamycinhydrochlorid, Daptomycin und Octenidindihydrochlorid und von Gentamicinsulfat hergestellt. Nach Vermischung dieser Pasten B mit der Paste A1 im Gewichtsverhältnis von 1 : 1 zeigten die gemischten Pasten C ein ähnliches Aushärtungsverhalten wie die (nicht erfindungsgemäße) Kombination der Paste A6 mit der Paste B6 im Gewichtsverhältnis von 1 : 1.

Zusätzlich wurden auch Pasten unter Verwendung von Bariumsulfat anstelle von Zirkoniumdioxid hergestellt. Diese Pasten hatten ein ähnliches Aushärtungsverhalten wie die aus den Pasten A1-6 und B1-6 hergestellten Pasten C1-6.

Es wurden weiterhin auch Pasten A analog dem Beispiel A1 unter Verwendung von t-Butyl-hydroperoxid, t-Amyl-hydroperoxid und Dicumyl-peroxid anstelle von Cumol-hydroperoxid hergestellt. Nach Vermischung dieser Pasten A mit der Paste B1 im Gewichtsverhältnis von 1 : 1 zeigten die gemischten Pasten ein ähnliches Verhalten wie die Kombination der Pasten A1 mit der Paste B1.

## Patentansprüche

1. Knochenzement-Kit, aufweisend eine Paste A und eine Paste B, wobei
(a) Paste A
(a1) wenigstens ein radikalisch polymerisierbares Monomer,
(a2) wenigstens ein in (a1) lösliches Polymer in einem Bereich von 3 bis 16 Gew.-% in Bezug auf das Gesamtgewicht der Paste, und
(a3) wenigstens einen Polymerisationsinitiator aus der Gruppe der Peroxide in einer Menge in einem Bereich von 0,01 bis 10 Gew.-%, bezogen auf das Gesamtgewicht der Paste A beinhaltet;
(b) Paste B
(b1) wenigstens ein radikalisch polymerisierbares Monomer,
(b2) wenigstens ein in (b1) lösliches Polymer in einem Bereich von 25 bis 85 Gew.-% in Bezug auf das Gesamtgewicht der Paste, und
(b3) wenigstens einen Polymerisationsbeschleuniger beinhaltet;
und wobei mindestens eine der Pasten A und B als Komponente (a4) bzw. (b4) wenigstens einen in dem wenigstens einen radikalisch polymerisierbaren Monomer (a1) bzw. (b1) schwer- oder unlöslichen Füllstoff beinhaltet, wobei der Füllstoff ein partikulärer anorganischer Füllstoff mit einer BET-Oberfläche von mindestens 40 m²/g ist und wobei Paste A 1 bis 50 Gew.-%, bezogen auf das Gesamtgewicht der Paste A, des Füllstoffs (a4) und Paste B weniger als 5 Gew.-%, bezogen auf das Gesamtgewicht der Paste B, des Füllstoffs (b4) beinhaltet.

2. Kit nach Anspruch 1, wobei der partikuläre anorganische Füllstoff an den Partikeln kovalent gebundene Hydroxylgruppen aufweist.

3. Kit nach Anspruch 2, wobei der partikuläre anorganische Füllstoff an den Partikeln kovalent gebundene HO-Si-Gruppen (Silanol-Gruppen) aufweist.

4. Kit nach Anspruch 3, wobei der partikuläre anorganische Füllstoff pyrogenes Siliziumdioxid mit einer BET-Oberfläche von mindestens 200 m²/g ist.

5. Kit nach Anspruch 4, wobei der partikuläre anorganische Füllstoff pyrogenes Siliziumdioxid mit einer BET-Oberfläche von mindestens 300 m²/g ist.

6. Kit nach einem der vorhergehenden Ansprüche, wobei das radikalisch polymerisierbare Monomer (a1) bzw. (b1) ein Methacrylsäureester ist.

7. Kit nach einem der vorhergehenden Ansprüche, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

8. Kit nach einem der vorhergehenden Ansprüche, wobei das in (a1) bzw. (b1) lösliche Polymer (a3) bzw. (b3) ausgewählt ist aus der Gruppe bestehend aus Poly (methacrylsäuremethylester), Poly (methacrylsäureethylester), Poly(methylmethacrylsäurepropylester), Poly (methacrylsäureisopropylester), Poly(methylmethacrylat-co-methylacrylat), Poly(styrol-co-methylmethacrylat) und einer Mischung aus mindestens zwei dieser Polymere.

9. Kit nach einem der vorhergehenden Ansprüche, wobei der wenigstens eine Polymerisationsinitiator (a3) ausgewählt ist aus der Gruppe bestehend Cumol-hydroperoxid, 1,1,3,3-Tetramethylbutylhydroperoxid, t-Butyl-hydroperoxid, t-Amyl-hydroperoxid, Di-Isopropylbenzen-monohydroperoxid und einer Mischung aus mindestens zwei davon.

10. Kit nach einem der Ansprüche 1 bis 9, wobei der wenigstens eine Polymerisationsbeschleuniger (b3) ausgewählt ist aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en, 1,5-Diazabicyclo(4.3.0)non-5-en, Phthalimid, Maleimid, Succinimid, Pyromelithsäurediimid und eine Mischung aus mindestens zwei davon.

11. Kit nach Anspruch 10, wobei die Paste B den Polymerisationsbeschleuniger (b3) in einer Menge in einem Bereich von 0,1 bis 10 Gew.-%, bezogen auf das Gesamtgewicht der Paste B, beinhaltet.

12. Kit nach einem der vorhergehenden Ansprüche, wobei die Paste B als Polymerisationsbeschleuniger (b3) eine Kombinationen aus Schwermetallsalzen und mindestens einem Vertreter aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)-non-5-en, Phthalimid, Maleimid, Succinimid und Pyromelithsäurediimid beinhaltet.

13. Kit nach einem der vorhergehenden Ansprüche, wobei die Paste A wenigstens einen Copolymerisationsbeschleuniger (a5) beinhaltet, der ausgewählt ist aus der Gruppe bestehend aus N,N-Dimethyl-p-toluidin, N,N-Bis-hydroxyethyl-p-toluidin, N,N-Dimethyl-anilin, 1,8-Diazabicyclo[5.4.0]-undec-7-en, 1,5-Diazabi-cyclo(4.3.0)non-5-en und einer Mischung aus mindestens zwei davon.

14. Kit nach Anspruch 13, wobei die Paste A den wenigstens einen Copolymerisationsbeschleuniger (a5) in einer Menge in einem Bereich von 0,1 bis 10 Gew.-%, bezogen auf das Gesamtgewicht der A, beinhaltet.

15. Kit nach einem der vorhergehenden Ansprüche, wobei die Pasten A und/oder B den Füllstoff (a4) bzw. (b4) in einer solchen Menge enthalten, das in der durch Vermischen der Pasten A und B hergestellten Paste C der Füllstoff in einer Menge in einem Bereich von 0,5 bis 25,0 Gew.-% enthalten ist.

16. Formkörper, erhältlich durch die Polymerisation einer Paste hergestellt aus dem in den Ansprüchen 1 bis 15 definierten Kit.

## Claims

1. A bone cement kit, comprising a paste A and a paste B, wherein
(a) paste A contains
(a1) at least one radically polymerisable monomer,
(a2) at least one polymer that is soluble in (a1) and is within a range from 3 to 16 weight per cent in relation to the total weight of the paste, and
(a3) at least one polymerisation initiator from the group of peroxides in a quantity within a range from 0.01 to 10 weight per cent in relation to the total weight of paste A;
(b) paste A contains
(b1) at least one radically polymerisable monomer,
(b2) at least one polymer that is soluble in (b1) and is within a range from 25 to 85 weight per cent in relation to the total weight of the paste, and
(b3) at least one polymerisation accelerator;
and wherein at least one of the pastes A and B contains as a component (a4) or (b4), respectively, at least one filler that is hardly soluble or insoluble in the at least one radically polymerisable monomer (a1) or (b1), respectively, wherein the filler is a particulate inorganic filler with a BET surface of at least 40 m²/g and wherein, in relation to the total weight of paste A, paste A contains 1 to 50 weight per cent of the filler (a4) and, in relation to the total weight of paste B, paste B contains less than 5 weight per cent of the filler (b4).

2. The kit in accordance with Claim 1, wherein the particulate inorganic filler comprises hydroxyl groups covalently bonded to the particles.

3. The kit in accordance with Claim 2, wherein the particulate inorganic filler comprises HO-Si groups (silanol groups) covalently bonded to the particles.

4. The kit in accordance with Claim 3, wherein the particulate inorganic filler is pyrogenic silicon dioxide having a BET surface of at least 200 m²/g.

5. The kit in accordance with Claim 4, wherein the particulate inorganic filler is pyrogenic silicon dioxide having a BET surface of at least 300 m²/g.

6. The kit in accordance with any one of the preceding claims, wherein the radically polymerisable monomers (a1) and (b1), respectively, are methyl methacrylates.

7. The kit in accordance with any one of the preceding claims, wherein paste A and paste B contain the at least one radically polymerisable monomer (a1) and (b1), respectively, in a quantity within a range from 15 to 85 weight per cent, each in relation to the total weight of paste A and paste B, respectively.

8. The kit in accordance with any one of the preceding claims, wherein the polymers (a3) and (b3), respectively, are selected from the group consisting of poly(methyl methacrylate), poly(ethyl methacrylate), poly(methyl propyl methacrylate), poly(isopropyl methacrylate), poly(methyl methacrylate co-methyl acrylate), poly(styrol-co-methyl methacrylate) and a mixture consisting of at least two of these polymers.

9. The kit in accordance with any one of the preceding claims, wherein the at least one polymerisation initiator (a3) is selected from the group consisting of cumene hydroperoxide, 1,1,3,3-tetramethylbutyl hydroperoxide, tert-butyl hydroperoxide, tert-amyl hydroperoxide, diisopropyl benzene monohydroperoxide and a mixture consisting of at least two thereof.

10. The kit in accordance with any one of Claims 1 to 9, wherein the at least one polymerisation accelerator (b3) is selected from the group consisting of N,N-dimethyl-p-toluidine, N,N-bis-hydroxyethyl-p-toluidine, N,N-dimethylaniline, methyltrioctylammonium chloride, tetrabutylammonium chloride, lithium chloride, saccharin, 1,8-diazabicyclo[5.4.0]undec-7-ene, 1,5-diazabicyclo(4.3.0)non-5-ene, phthalimide, maleimide, succinimide, pyromellitic acid diimide and a mixture consisting of at least two thereof.

11. The kit in accordance with Claim 10, wherein paste B contains the polymerisation accelerator (b3) in a quantity within a range from 0.1 to 10 weight per cent in relation to the total weight of paste B.

12. The kit in accordance with any one of the preceding claims, wherein paste B, as the polymerisation accelerator (b3), contains a combination of heavy metal salts and at least one representative of the group consisting of N,N-dimethyl-p-toluidine, N,N-bis-hydroxyethyl-p-toluidine, N,N-dimethylaniline, methyltrioctylammonium chloride, tetrabutylammonium chloride, lithium chloride, saccharin, 1,8-diazabicyclo[5.4.0]undec-7-ene, 1,5-diazabicyclo(4.3.0)non-5-ene, phthalimide, maleimide, succinimide, and pyromellitic acid diimide.

13. The kit in accordance with any one of the preceding claims, wherein paste A contains at least one copolymerisation accelerator (a5) which is selected from the group consisting of N,N-dimethyl-p-toluidine, N,N-bis-hydroxyethyl-p-toluidine, N,N-dimethylaniline, 1,8-diazabicyclo[5.4.0]undec-7-ene, 1,5-diazabicyclo(4.3.0)non-5-ene and a mixture consisting of at least two thereof.

14. The kit in accordance with Claim 13, wherein paste A contains the at least one copolymerisation accelerator (a5) in a quantity within a range from 0.1 to 10 weight per cent in relation to the total weight of paste A.

15. The kit in accordance with any one of the preceding claims, wherein pastes A and/or B contain the filler (a4) and (b4), respectively, in such a quantity that paste C, which is produced by mixing pastes A and B, contains the filler in a quantity within a range from 0.5 to 25.0 weight per cent.

16. A mould, obtainable by polymerisation of a paste produced from the kit defined in Claims 1 to 15.

## Revendications

1. Kit de ciment osseux, présentant une pâte A et une pâte B,
(a) la pâte A contenant
(a1) au moins un monomère polymérisable de manière radicalaire,
(a2) au moins un polymère soluble dans (al), de l'ordre de 3 à 16 % en poids par rapport au poids total de la pâte et
(a3) au moins un initiateur de polymérisation du groupe des peroxydes dans une quantité de l'ordre de 0,01 à 10 % en poids en ce qui concerne le poids total de la pâte A ;
(b) la pâte B contenant
(b1) au moins un monomère polymérisable de manière radicalaire,
(b2) au moins un polymère soluble dans (b1), de l'ordre de 25 à 85 % en poids par rapport au poids total de la pâte et
(b3) au moins un accélérateur de polymérisation ;
et au moins l'une des pâtes A et B comme composants (a4) et/ou (b4) contenant au moins une charge difficilement soluble ou insoluble dans l'au moins un monomère polymérisable de manière radicalaire (a1) et/ou (b1), la charge étant une charge de particules inorganiques avec une surface BET d'au moins 40 m²/g et la pâte A contenant 1 à 50 % en poids de la charge (a4) par rapport au poids total de la pâte A et la pâte B contenant moins de 5% en poids de la charge (b4) par rapport au poids total de la pâte B.

2. Kit conformément à la revendication n°1, la charge de particules inorganiques présentant des groupes d'hydroxyle liés de manière covalente aux particules.

3. Kit conformément à la revendication n°2, la charge de particules inorganiques présentant des groupes HO-Si (groupes silanol) liés de manière covalente aux particules.

4. Kit conformément à la revendication n°3, la charge de particules inorganiques étant du dioxyde de silicium pyrogéné avec une surface BET d'au moins 200 m²/g.

5. Kit conformément à la revendication n°4, la charge de particules inorganiques étant du dioxyde de silicium pyrogéné avec une surface BET d'au moins 300 m²/g.

6. Kit conformément à l'une des revendications précédentes, le monomère polymérisable de manière radicalaire (a1) et/ou (b1) étant un ester d'acide méthacrylique.

7. Kit conformément à l'une des revendications précédentes, la pâte A et la pâte B contenant au moins un monomère polymérisable de manière radicalaire (a1) et/ou (b1) dans une quantité de l'ordre de 15 à 85 % en poids par rapport au poids total de la pâte A et/ou de la pâte B.

8. Kit conformément à l'une des revendications précédentes, le polymère (a3) et/ou (b3) soluble dans (a1) et/ou (b1) étant sélectionné dans le groupe composé de poly(ester méthylique d'acide méthacrylique), poly(ester éthylique d'acide méthacrylique), poly(ester propylique d'acide méthylméthacrylique), poly(ester isopropylique d'acide méthacrylique), poly(méthylméthacrylate-co-méthylacrylate), poly(styrène-co-méthylméthacrylate) et d'un mélange d'au moins deux de ces polymères.

9. Kit conformément à l'une des revendications précédentes, au moins un initiateur de polymérisation (a3) étant sélectionné dans le groupe composé de hydroperoxyde de cumène, 1,1,3,3-hydroperoxyde tétraméthylbutylique, hydroperoxyde de t-butyle, hydroperoxyde de t-amyle, mono-hydroperoxyde de diisopropylbenzène et d'un mélange d'au moins deux de ces derniers.

10. Kit conformément à l'une des revendications n°1 à n°9, l'au moins un accélérateur de polymérisation (b3) étant sélectionné dans le groupe composé de N,N-diméthyl-p-toluidine, N,N-bis-hydroxyéthyl-p-toluidine, N,N-diméthyl-aniline, chlorure d'ammonium trioctylméthylique, chlorure d'ammonium tétrabutylique, chlorure de lithium, saccharine, 1,8-diazabicyclo[5.4.0]undec-7-en, 1,5-diazabicyclo[4.3.0]non-5-en, phthalimide, maléimide, succinimide, diimide d'acide pyromélithique et d'un mélange d'au moins deux de ces derniers.

11. Kit conformément à la revendication n° 10,
la pâte B contenant l'accélérateur de polymérisation (b3) dans une quantité de l'ordre de 0,1 à 10 % en poids par rapport au poids total de la pâte B.

12. Kit conformément à l'une des revendications précédentes, la pâte B en tant qu'accélérateur de polymérisation (b3) contenant une combinaison de sels de métaux lourds et au moins un représentant du groupe composé de N,N-diméthyl-p-toluidine, N,N-bis-hydroxyéthyl-p-toluidine, N,N-diméthyl-aniline, chlorure d'ammonium trioctylméthylique, chlorure d'ammonium tétrabutylique, chlorure de lithium, saccharine, 1,8-diazabicyclo[5.4.0]undec-7-en et 1,5-diazabicyclo[4.3.0]non-5-en, phthalimide, maléimide, succinimide et diimide d'acide pyromélithique.

13. Kit conformément à l'une des revendications précédentes, la pâte A contenant au moins un accélérateur de copolymérisation (a5) qui est sélectionné dans le groupe composé de N,N-diméthyl-p-toluidine, N,N-bis-hydroxyéthyl-p-toluidine, N,N-diméthyl-aniline, 1,8-diazabicyclo[5.4.0]undec-7-en, 1,5-diazabicyclo[4.3.0]non-5-en et d'un mélange d'au moins deux de ces derniers.

14. Kit conformément à la revendication n°13, la pâte A contenant au moins un accélérateur de copolymérisation (a5) dans une quantité de l'ordre de 0,1 à 10 % en poids par rapport au poids total de la pâte A.

15. Kit conformément à l'une des revendications précédentes, les pâtes A et/ou B contenant la charge (a4) et/ou (b4) dans une quantité telle que la charge dans une quantité de l'ordre de 0,5 à 25,0 % en poids soit contenue dans la pâte C produite par le mélange des pâtes A et B.

16. Corps moulé obtenu par la polymérisation d'une pâte produite à partir du kit défini dans les revendications n°1 à n°15.
